# EUROPEAN PATENT SPECIFICATION

(11) **EP 2 194 970 B1**
(45) Date of publication and mention of the grant of the patent: **02.09.2015**
(21) Application number: 08789190.9
(22) Date of filing: 04.07.2008
(51) Int. Cl.: A61K 9/107, A61K 38/17, A61K 47/12, A61K 47/14, A61K 31/201, A61K 31/203, A61K 38/00, A61K 38/02, A61K 38/04, A61K 38/16

(54) **ENHANCEMENT OF THE EFFICACY OF THERAPEUTIC PROTEINS**
VERSTÄRKUNG DER WIRKSAMKEIT THERAPEUTISCHER PROTEINE
AMÉLIORATION DE L'EFFICACITÉ DE PROTÉINES THÉRAPEUTIQUES

(30) Priority: 05.07.2007 ZA 200705497
(43) Date of publication of application: 16.06.2010
(73) Proprietor: The North West University, Potchefstroom, 2531 (ZA)
(72) Inventor: GROBLER, Anne Frederica, 2531 Potchefstroom (ZA); KOTZE, Abraham Frederik, 2531 Potchefstroom (ZA); DU PLESSIS, Jeanetta, 2531 Potchefstroom (ZA)
(74) Representative: Bates, Philip Ian
(86) International application number: PCT/IB2008/052692
(87) International publication number: WO 2009/004595

(56) References cited:
- WO-A-02/05849
- WO-A-02/05850
- WO-A-97/17978
- WO-A-2007/096833
- SCHRODER U ET AL: "Nasal and parenteral immunizations with diphtheria toxoid using monoglyceride/fatty acid lipid suspensions as adjuvants" VACCINE, BUTTERWORTH SCIENTIFIC. GUILDFORD, GB, vol. 17, no. 15-16, 9 April 1999 (1999-04-09), pages 2096-2103, XP004165061 ISSN: 0264-410X

## Description

### FIELD OF THE INVENTION

This invention relates generally to the field of drug administration, more particularly to the oral, nasal, topical or parenteral delivery of peptide or protein drugs by entrapment into a fatty acid (hereinafter also referred to as FA) based nitrous oxide saturated matrix in the form of a vesicles or microsponges. The invention further relates to the enhancement in the efficacy of protein or peptide drugs by its entrapment into the fatty acid-based vesicles and microsponges of the invention. In addition, the invention relates to an increase in the therapeutic window of the administered protein or peptide drug

### DEFINITIONS AND BACKGROUND OF THE INVENTION

Peptides and proteins are both composed of amino acid residues linked together by amide or peptide bonds. The distinction between these two classes of compounds is based on different conventions, none of which is universally satisfactory. The terms protein and peptide will accordingly be used interchangeably in this specification to signify compounds that contain multiple amino acid residues linked by amide bonds.

As used herein, "protein" is not limited to native (i.e., naturally-occurring) or full-length proteins, but is meant to encompass protein fragments having a desired activity or other desirable biological characteristic, as well as mutants or derivatives of such proteins or protein fragments that retain a desired activity or other biological characteristic. Mutant proteins encompass proteins having an amino acid sequence that is altered relative to the native protein from which it is derived, where the alterations can include amino acid substitutions (conservative or non-conservative), deletions, or additions (e.g., as in a fusion protein). Derivatives of proteins include proteins that have been modified by the binding of other molecules such as carbohydrates to the protein. Reference to "peptide" herein is intended to have a corresponding meaning.

Also in this specification the expressions "therapeutic mammalian protein" and "therapeutic mammalian peptide" when used in the context of the invention to be disclosed herein are intended to signify proteins or peptides (as qualified above) which, in their naturally-occuring form are produced by a mammalian body and which have therapeutic properties when administered to a mammal, and are thus intended to exclude proteins and peptides which are produced by micro-organisms such as proteins and peptides that have antigenic properties and may thus be used in the preparation of vaccines, and also to exclude salmon calcitonin and human growth hormone as well as any protein or peptide agent specifically named in WO9717978 in respect of the invention entitled ADMINISTRATION MEDIA FOR ANALGESIC, ANTI-INFLAMMATORY AND ANTI-PYRETIC DRUGS CONTAINING NITROUS OXIDE AND PHARMACEUTICAL COMPOSITIONS CONTAINING SUCH MEDIA AND DRUGS, or in WO0205850 in respect of the invention entitled ENHANCEMENT OF THE ACTION OF ANTI-INFECTIVE AGENTS (including ramoplanin, teicoplanin, vancomycin and interferon alpha), or in WO0205851 in respect of the invention entitled ENHANCEMENT OF THE ACTION OF CENTRAL AND PERIPHERAL NERVOUS SYSTEM AGENTS, and is further also to exclude proteins and peptides that are incorporated in a dosage form for the purpose of targeting a specific receptor to which any therapeutically active agent also present in such dosage form (or precursor of such agent or nucleic acid substance coding for such agent) is intended to be delivered. The aforementioned WO patent publications are accordingly incorporated by reference in this description.

The proteins with which this invention is specifically concerned is the group consisting of insulin, parathyroid hormone, parathyroid-like hormone, glucagon, insulinotrophic hormone, vasopressin and hormones involved in the reproductive system, chemotactins; cytokines including interleukins 1,2 and RA but excluding the interferons; chemokines; enzymes including proteases and protease inhibitors; growth factors including acidic and basic fibroblast growth factors, epidermal growth factor, tumor necrosis factors, platelet derived growth factor, granulocyte macrophage colony stimulating factor, neurite growth factor and insulin-like growth factor-1, hormones including the gonadotrophins and somatomedians, immunoglobulins, lipid-binding proteins and soluble CD4, urokinase, streptokinase, superoxide dismutase (SOD), catalase, phenylalanine ammonia lyase, L-asparaginase, pepsin, uricase, trypsin, chymotrypsin, elastase, carboxypeptidase, lactase, sucrase, ciliary neurite transforming factor (CNTF), clotting factor VIII, erythropoietin, thrombopoietin, insulintropin, cholecystokinin, glucagon-like peptide I, intrinsic factor, Ob gene product, tissue plasminogen activator (tPA), brain-derived neurite factor, phenylalanine transporter (for phenylketonuria), brush border enzymes and transporters.

Proteins are essential to virtually all biological functions, including metabolism, growth, reproduction, and immunity. As such, they have a potential role as pharmaceutical agents for the treatment of a wide range of human diseases. Indeed, they have already been used to treat diseases such as cancer, hemophilia, anemia and diabetes successfully, and for a number of diseases is the only effective treatment. Because many congenital and acquired medical disorders result from inadequate production of various gene products, protein or peptide therapy, such as hormone replacement therapy, provides a means to treat these diseases through their supplementation to the patient. As with almost all therapies, the therapy that is most easily administered, least expensive, and most likely to realize patient compliance is the therapy of choice.

Although protein drugs have enormous therapeutic potential, their more widespread use has been limited by several restrictive technical factors. These include the following considerations:
Proteins remain difficult and expensive to manufacture compared to other pharmaceuticals. Large-scale purification of proteins in bioactive form can be a limiting step in the commercialization of these drugs. The production of these drugs may be cost prohibitive in developing countries.
Many proteins are metabolized in the body, resulting in a short circulating half life and a need for frequent dosing.
Due to the hydrophilic nature and molecular size of protein drugs they are poorly absorbed across mucosal epithelia, both transcellularly and paracellularly, leading to poor bioavailability.
Proteins are often degraded in the harsh gastric environment after oral administration. Protection against degradation may make the *peroral* administration of these drugs viable.
Clearance of proteins is generally fast, and they are eliminated quickly in the patient. This results in the need for frequent re-administration, contributing to cost. An increase in the circulating half life of the peptide at therapeutic concentrations would therefore be advantageous.
Generally, protein drugs must be given by injection. This increases the complexity and expense of the treatment. The disagreeable nature of administration also limits potential clinical applications and decrease patient compliance.

An enhancement in either the absorption, or the efficacy of these drugs should accordingly contribute to more cost effective and hence affordable peptide or protein drugs.

Administration of therapeutic protein products (such as hormones, growth factors, signaling molecules, neurotransmitters, cytokines or polypeptides for protein replacement therapy) by administration routes other than the parenteral route has attracted wide attention as a method to treat various mammalian diseases. Due to the described problems with other administration routes, it is necessary to employ a drug delivery system or penetration enhancer for administration of these drugs via alternative administration routes. Poor bioavailability may be partly overcome by the inclusion of absorption enhancers in protein drug formulations although that is not necessarily the best solution.

With the advent of Human Genome Organisation (HUGO) new proteins are discovered at an increasing pace, and known proteins become available as therapeutic agents. New ways and methods to expand the application of these molecules as drugs by improving the feasibility and convenience of their use are now required. The present invention addresses precisely such an effort.

The oral route is the most common, simple, convenient and physiological way of administering traditional active compounds. The oral route generally does not lend itself to the administration of protein drugs due to the problems described above. A variety of delivery systems have been developed to try to accomplish therapeutic *peroral* delivery of proteins (for reviews, see Chang et al. 1994 Gastroenterol. 106: 1076-84; Morsey et al. 1993 JAMA 270: 2338-45; and Ledley 1992 J. Pediatr. Gastroenterol. Nutr. 14: 328-37).

The ease of administration via an oral or other lumenal route, or the nasal or topical route allows administration of peptides through non-invasive procedures. These routes share a number of significant physiological characteristics:
(a) The administration sites can be regarded as existing "outside" of the body as the anatomical receptacle of the drug is separated from the circulation by a continuous biological barrier.
(b) The sites are separated from the circulation by (a) continuous layer(s) of cells; in the case of oral administration the intestinal epithelium, in the case of nasal administration the nasal epithelium and underlying layers and in the case of topical administration the epidermis and to some extent the dermis. The layer(s) thus form a continuous biological barrier with various levels of penetratability.
(c) Any drug administered thus remains in the exterior space, and cannot enter the body proper and its bloodstream, unless it first crosses the described cell layer(s). However, once the drug is absorbed into the epithelial cells of the nasal tract, or GI lumen, or has penetrated through the epidermis, it can be transported into the bloodstream where the therapeutic protein will presumably act in the same manner as current, injectable forms of the drug.
(d) The cells lining the biological barriers described all secrete a fluid (i.e. mucus or sweat) that may interfere with the stability or complicate absorption of the drug. Proteases present in such fluid may in each case cause degradation of the protein.

Thus past efforts to administer proteins through the oral, nasal or topical route have met with severe obstacles. Many of the existing delivery systems either have their own inherent drawbacks or are not entirely suitable for protein delivery. Moreover, delivery of the drugs via the bloodstream of the individual results in exposure of the proteins and any carrier associated with it to the immune system, which can result in immunological adverse reactions.

### The therapeutic mammalian protein: insulin

Insulin therapy is still the mainstay of the treatment of Type 1 and 2 diabetes and is the most widely used protein drug. Despite advances, it is still administered by subcutaneous injection or microneedles which cause disruption of the skin. Subcutaneous or microneedle administration suffers from disadvantages such as time lag between peak insulin levels and postprandial glucose levels, hypoglycemia, weight gain, peripheral hyperinsulineamia and poor patient compliance. An overdose of insulin may cause secondary effects such as the release of glucagon, growth hormone, catecholamines and corticosteroids as a result of pronounced hypoglycemia. Efforts to develop dosage forms that may circumvent or at the very least decrease these problems are ongoing.

Insulin is normally synthesized as pro-insulin by the β-cells of the islets of Langerhans found in the endocrine pancreas. In its processed form, it consists of an A and B chain with a combined a molecular weight of 5807.7 and an amino acid number of 51. Insulin is released from secretory granules in the β-cells of the islets directly into the blood stream at a low basal rate. A variety of stimuli, such as glucose, sugars, certain amino acids and vagal activity stimulates release of insulin. Under normal fasting conditions, the pancreas secretes about 40µg (1 IU) of insulin per hour into the hepatic portal vein. The insulin concentration of portal blood averages between 2 - 4 ng/ml, and the peripheral blood 0.5 ng/ml (12 µIU/ml). The plasma half-life of insulin is around 5 to 6 minutes in healthy people, with the degradation of insulin occurring mainly in the liver, kidneys and muscle. It is estimated that 50% of the insulin that reaches the liver by the hepatic portal vein is degraded and does not reach the general circulation.

Commercially available insulin preparations that are currently available can mostly be classed as:
- Ultra-short-acting insulin, with fast onset and short duration of action;
- Short-acting insulin, with fast onset of action;
- Intermediate acting insulin, and
- Long-acting insulin, with slow onset and a longer duration of action.

All of the above preparations are stabilized by the addition of zinc and/or protamines. Conventional subcutaneous insulin therapy mainly consists of split-dose injections of mixtures of short-acting and intermediate-acting preparations with the addition of long-acting insulin for prolonged duration of action to sustain overnight basal levels.

The oral route Is attractive for insulin therapy because of both pragmatic and physiological reasons. In practice, it Is associated with simplicity and comfort. Besides the discomfort of injections, the reuse of needles carries a risk of infection. Oral preparations are generally cheaper to manufacture, as they do not have to be sterile. A physiological advantage lies in the fact that it mimics the endogenous secretion of insulin more closely: Insulin Is absorbed from the intestine and reaches the liver via the hepatic portal vein, with a direct effect on the hepatic glucose production and the maintenance of energy levels by the liver, avoiding In this fashion hyperinsulinemic effecs Insulin administered parenterally on the other hand, does not simulate the normal dynamics of endogenous insulin secretion Despite these advantages of peroral insulin, this route has not been used successfully, as less than 0 5% of the orally administered dose is absorbed from the GI tract and less than 0 1% reaches the central bloodstream intact The oral delivery of complex drug molecules such as hormones Is currently receiving attention, with an interest in increasing the intestinal permeability of such large molecules and molecules with known poor bioavailability.

WO0205849 refers to a method for the administration of a nucleic acid substance to the cells of an animal, a plant or a micro-organism.

WO9717978 refers to novel preparations of analgesic, anti-inflammatory and antl-pyretlc drugs, and the enhancement of the efficacy of such agents.

WO0205850 refers to pharmaceutical preparations for use in combating infective organisms afflicting the animal body.

WO2007096833 refers to a plant supporting formulations.

### OBJECT OF THE INVENTION

A primary object of the present invention is to provide a method of administration of a therapeutic mammalian proteins as herein defined, and certain named proteins, through non-invasive means The primary object Is extended to provide a method whereby the efficacy of the administered therapeutic mammalian proteins, and certain named proteins, is enhanced and the amount of expensive active drug needed is reduced.

A secondary object is the stabilization of a therapeutic mammalian proteins, and certain named proteins, against degradation by a) masking of the protein against protease action and b) by the concomitant incorporation of protease inhibitor as hereinafter described The present invention is advantageous In that It may be used to protect therapeutic mammalian proteins drugs, and certain named protein drugs from enzyme action.

### DESCRIPTION OF THE INVENTION

According to the present invention there is provided a formulation for use in administration of at least one therapeutic mammalian protein to a mammal, and for enhancing the absorption, distribution and release of the at least one therapeutic mammalian protein in or on the mammal, the formulation consisting of at least one therapeutic mammalian protein in a micro-emulsion which micro-emulsion is constituted by a dispersion of vesicles or microsponges of a fatty acid based component in an aqueous or other pharmacologically acceptable carrier in which nitrous oxide is dissolved, the fatty acid based component comprises at least one long chain fatty acid based substance selected from the group consisting of free fatty acids and derivatives of free fatty acids, which derivatives thereof are selected from the group consisting of the C1 to C6 alkyl esters thereof, the glycerol-polyethylene glycol esters thereof, and the reaction product of hydrogenated and unhydrogenated natural oils composed largely of ricinoleic acid based oils with ethylene oxide.

The invention also provides for a formulation for use for the administration to a mammal of at least one protein wherein the at least one protein is selected from the group consisting of insulin, parathyroid hormone, parathyroid-like hormone, glucagon, insulinotrophic hormone, vasopressin and hormones involved.in the reproductive system, chemotactins; cytokines including interleukins 1 ,2 and RA but excluding the interferons; chemokines; enzymes including proteases and protease inhibitors; growth factors including acidic and basic fibroblast growth factors, epidermal growth factor, tumor necrosis factors, platelet derived growth factor, granulocyte macrophage colony stimulating factor, neurite growth factor and insulin-like growth factor-1, hormones including the gonadotrophins and somatomedians, immunoglobulins, lipid-binding proteins and soluble CD4, urokinase, streptokinase, superoxide dismutase (SOD), catalase, phenylalanine ammonia lyase, L-asparaginase, pepsin, uricase, trypsin, chymotrypsin, elastase, carboxypeptidase, lactase, sucrase, ciliary neurite transforming factor (CNTF), clotting factor VIII, erythropoietin, thrombopoietin, insulintropin, cholecystokinin, glucagon-like peptide I, intrinsic factor, Ob gene product, tissue plasminogen activator (tPA), brain-derived neurite factor, phenylalanine transporter, brush border enzymes and transporters, and for enhancing the absorption, distribution and release of the at least one protein in or on the mammal, the formulation consisting of the at least one protein in a micro-emulsion which micro-emulsion is constituted by a dispersion of vesicles or microsponges of a fatty acid based component in an aqueous or other pharmacologically acceptable carrier in which nitrous oxide is dissolved, the fatty acid based component comprising at least one long chain fatty acid based substance selected from the group consisting of free fatty acids and derivatives of free fatty acids.

The vesicles or microsponges used in the present invention are designed so as to enhance therapeutic mammalian protein, or above named protein, absorption and therapeutic mammalian protein, or above named protein, systemic circulation time while at the same time decreasing therapeutic mammalian protein, or above named protein, degradation. This combination of necessity results in increased efficacy of the therapy.

Non-invasive routes of administration such as per oral, topical or nasal routes require that any therapeutically active compound must first cross a continuous biological barrier consisting of a layer or layers of cells and sometimes some additional fibrous tissue before it can enter the body proper and its bloodstream. As described above, in this invention therapeutic mammalian protein, or above named protein, drugs are packaged into or entrapped within FA-based nitrous oxide saturated particles. Changes in the composition of the FA result in different types of particles, of which at least two types will specifically be addressed in the examples stated below.

Preferably, the composition also contains the antioxidant dl-α-tocopherol or a stable derivative of this antioxidant. Thus, the vehicle may contain α-tocopherol or one of its derivatives at a concentration of no less than 0.1 % and no more than 5% in addition to commercially available antioxidants. For example, the formulation can include one or more anti-oxidants, such as TBHQ (tert-butyl hydro quinone), BHA (butylated hydroxyanisole) or BHT (butylated hydroxytoluene), which can increase the degree of enhancement of the therapeutic mammalian protein, or above named protein, of interest, particularly where the stability of the drug molecule is at risk.

For purposes of increased shelf life, the composition may also contain protease inhibitors which are commercially available, such as bestatin.

The dispersion is preferably characterized in that at least 50% of the vesicles are of a diametrical size of between 80 nanometer and 3µm and that of the microsponges between 1.5 and 6.0µm. Both the size and shape of the vesicles are reproducible. It will be understood that the vesicles or microsponges in the dispersion are elastic and not necessarily of perfectly spherical shape and accordingly the term "diametrical size" is not to be understood as a term of geometric precision. It is further to be understood that it is not practicable to determine such diametrical size in three dimensions without the use of highly sophisticated instrumentation. It is accordingly to be determined in two dimensions by means of microscopic observation and thus refers to the maximum measurement across observed vesicles or microsponges as seen in two dimensions.

Various fatty acids and modified fatty acids (e g , ethylated fatty acids) can be used in accordance with the present invention. Techniques for the modification of the fatty acids are known in the art (Villeneuve et al; 2000 Journal of Molecular Catalysis: Enzymatic; 9 113-148, Demirbas A, 2007, Energy Conversion and Management; in press; available online at www.sciencedirect com). For example, polyethylene glycol (PEG) molecules, small peptides or carbohydrate molecules may be linked to the carboxyl group of the fatty acid. The modifications are preferred to be biologically functional and to support a desired characteristic. Some modified fatty acids are commercially available.

Preferably both fatty acids containing ethyl groups and polyethylene groups attached to their carbonyl groups are used. Numerous such modified fatty acids are known in the art and are commercially available. In general, each such commercial preparation consists of a variety of modified fatty acids.

The fatty acid based component may be selected from the group consisting of oleic acid, linoleic acid, alpha- linolenic acid, gamma-linolenic acid, arachidonic acid, eicosapentaenoic acid [C20:5ω3], decosahexaenoic acid [C22:6ω3], and ricinoleic acid, and derivatives thereof selected from the group consisting of the C₁ to C₆ alkyl esters thereof, the glycerol-polyethylene glycol esters thereof, and the reaction product of hydrogenated and unhydrogenated natural oils composed largely of ricinoleic acid based oils, such as castor oil, with ethylene oxide. In one form of the invention the fatty acid component of the micro-emulsion may consist or include a mixture of esterified fatty acids. In this regard use may be made of the commercially available product known as Vitamin F Ethyl Ester. Despite the availability of commercial products containing combinations of esterfied fatty acids, the fatty acid based component may be constituted from selected single fatty acids or modified fatty acids according to the cellular and subcellular target of the invention.

For microsponges, preferably very long chain polyunsaturated fatty acids are used. The long chain fatty acids may be selected from any of a variety of such fatty acids known in the art. The fatty acid component may thus alternatively include or consist of the long chain fatty acids known as eicosapentaenoic acid [C20:5w3] and decosahexaenoic acid [C22:6ω3]. Such a product combination is available from Roche under the trade name "Ropufa '30' n-3 oil". An alternative product that may be used for this purpose is one of the group of Incromega products available from Croda.

The fatty acid component may in addition to the aforementioned substances or mixtures of substances also include the reaction product of hydrogenated natural oils composed largely of ricinoleic acid based oils with ethylene oxide. It is preferable for this substance to be produced from castor oil of which the fatty acid content is known to be predominantly composed of ricinoleic acid. This product may be modified as to the extent of hydrogenation, ethylation and the addition of groups such as polyethylene glycol. A range of such products is being marketed by BASF under the trade description of Cremophor of various grades. According to a preferred form of the invention for certain applications, the ricinoleic acid molecules are modified by the addition thereto of polyethylene glycol groups which comprise between 35 and 45 ethylene oxide units.

The typical fatty acid profile of the FA-based vesicles is as follows:
0 .2324% of ethylated C_{16.0} fatty acids
0.098% of ethylated C_{18.0} fatty acids
0.6076% of ethylated C_{18.1} fatty acids
0.9744% of ethylated C_{18.2} fatty acids
0.784% of ethylated C_{18.4} fatty acids
1.00% of glycerol-polyethylene glycol esters of ricinoleic acid

The typical fatty acid profile of the FA-based microsponges is as follows:
0.2324% of ethylated C_{16.0} fatty acids
0.098% of ethylated C_{18.0} fatty acids
0.6076% of ethylated C_{18.1} fatty acids
0.9744% of ethylated C_{18.2} fatty acids
0.784% of ethylated C_{18.4} fatty acids
1.00% of glycerol-polyethylene glycol esters of ricinoleic acid
0.25% of ethylated C_{20.3} fatty acids
0.25% of ethylated C_{22.3} fatty acids

The vehicle further contains nitrous oxide gas dissolved in the fatty acid mixture to impart the requisite size distribution of vesicles and the requisite stability to the micro-emulsion. The nitrous oxide gas is sparged through the fatty acid phase or the water phase or the final formulation containing the therapeutic mammalian protein, or above named protein, of the present invention.

In its prefered form, the FA-based particles consist of an oil phase and a water phase, both of which are present in association with nitrous oxide. A precursor form of these particles, consisting of only the oil phase in association with nitrous oxide is generally used for oral applications. The aqueous phase may consist of sterile water or sterile buffers, depending on the properties of the drug to be entrapped, while the oil phase consists of a combination of modified fatty acids. The fatty acids are manipulated to ensure remarkably high entrapment capabilities, extremely fast rate of transport and cellular delivery.

There is provided a method for producing a delivery vehicle according to the present invention as defined above, comprising the steps of mixing the fatty acid based component with water to obtain a micro-emulsion, and introducing nitrous oxide gas into the mixture to impart the requisite size distribution of vesicles and the requisite stability to the micro-emulsion. Techniques for production of self-emulsifying micro-emulsions are known in the art (see, for example, Gursoy and Benita; Biomedicine & Pharmacotherapy, Volume 58, Issue 3, April 2004, Pages 173-182). In this regard, the mixing of the fatty acid component is preferably effected in the presence of heating, with stirring, preferably by means of a high speed shearer.

The therapeutic mammalian protein, or above named protein, drug may be pre-mixed with either the oil phase or the water phase, depending on the hydrophobicity and polarity of the specific therapeutic mammalian protein, or above named protein, to be entrapped. In this case, the mixing of the formulation is preferably effected after cooling the fatty acid component to below 50°C and with some stirring, preferably by means of a low speed shearer in the presence of the nitrous oxide gas. The mixing may also occur after the formation of the particles by gentle mixing.

The nitrous oxide gas may be introduced into the water either before or after the fatty acid based component of the micro-emulsion is mixed with the water. Thus, the nitrous oxide gas may be dissolved in the water to obtain a saturated solution of the nitrous oxide gas in water, and the saturated solution of the nitrous oxide gas is thereafter mixed with the fatty acid component of the micro-emulsion being prepared. The saturated solution of the nitrous oxide gas in water may be prepared by sparging the water with the nitrous oxide gas, or by exposing the water to the nitrous oxide gas at a pressure in excess of atmospheric pressure for a period of time in excess of the time required for the water to become saturated with the nitrous oxide gas. An emulsion of the fatty acid component in water may first be prepared and may thereafter be gassed by exposing the emulsion to the nitrous oxide gas. This is preferably done by sparging.

Formulations are typically available in forms that can be used in dosage devices or formulations used in oral, nasal or topical administrations. Such forms include any additives that further enhance effectiveness, stability, or ease of application such as penetration enhancers, thickeners and other adjuvants, and any other ingredients including solvents, carriers, or dyes. The application method and species to be treated determine which formulation is preferable.

This invention focuses on an effective method of transport of therapeutic mammalian proteins, or above named proteins across the biological barriers. The formulation comprising the therapeutic mammalian protein, or above named protein, of interest entrapped in a transport vehicle is absorbed into cells lining the anatomical receptacles (i.e. nasal cavity, GI lumen or skin) after being administered externally. Preferably, the therapeutic mammalian protein, or above named protein, of interest is stably entrapped in the transport vehicle. The therapeutic mammalian protein, or above named protein, is then transported to the systemic circulation, preferably in therapeutically effective amounts. Once in the circulation, therapeutic mammalian proteins, or above named proteins that serve as replacement or supplementation of therapeutic mammalian protein, or above named protein, therapy acts in the same manner as if they were naturally expressed by the subject. Furthermore, where the therapeutic mammalian protein, or above named protein, is an exogenous therapeutic mammalian protein, or above named protein, that provides a desired therapeutic effect the drug exhibits the same activity as if it were delivered by conventional injection methods.

Preferably, sufficient levels of the therapeutic mammalian protein, or above named protein, of interest are absorbed into the blood for therapeutic mammalian protein, or above named protein, therapy to be effective. The therapeutic effect of the therapeutic mammalian protein, or above named protein, may be enhanced by targeting of the fatty acid matrix through covalent binding of targeting ammo acid sequences, motifs or peptides to the carbonyl groups of the fatty acids, or by attaching other elements which mediate specific organ selection.

### Therapeutic mammalian proteins and conditions amenable to treatment by protein or peptide therapy

Preferably, the therapeutic mammalian protein, or above named protein, entrapped in the particles of the invention can be any therapeutic mammalian protein, or above named protein, that can be used for therapeutic mammalian protein, or above named protein, replacement or supplementation, be it caused by either an inherited or acquired disease associated with a specific therapeutic mammalian protein, or above named protein, deficiency. The aim of the intervention would be to restore the levels of the deficient therapeutic mammalian protein, or above named protein, to normal levels in at least the systemic circulation but preferably also in the applicable organs, tissue or cells. Conditions caused by therapeutic mammalian protein, or above named protein, deficiencies that can be treated by replacement or supplementation include diabetes, hemophilia, anemia, immunodeficiencies, nutrient absorption deficiencies, and steroid hormone replacements.

The therapeutic mammalian protein, or above named protein, may also be any therapeutic mammalian protein, or above named protein, that may regulate or switch on or switch off a specific pathway in the body. Numerous therapeutic mammalian proteins, or above named proteins that are desirable for protein therapy are well known in the art. Proteins commonly used in treatments can be delivered by various administration routes using the present invention. Such therapeutic mammalian proteins, or above named proteins are disclosed in, for example, the Physicians' Desk Reference (1994 Physicians' Desk Reference, 48th Ed., Medical Economics Data Production Co., Montvale, N.J.; incorporated by reference) and can be dosed using methods in Harrison's Principles of Internal Medicine and/or the AMA "Drug Evaluations Annual" 1993, all incorporated by reference.

Proteins can be either completely lacking or defective in which case complete replacement needs to be undertaken. Alternatively, the protein may be under-expressed in which case the invention would be used for supplementation therapy. A protein may also be over-expressed and therapy may need to supply a therapeutic mammalian protein, or above named protein, to either regulate or degrade the over-expressed protein.

Exemplary preferred therapeutic mammalian proteins, or above named proteins include the hormones and peptide hormones such as insulin, parathyroid hormone, parathyroid-like hormone, glucagon, insulinotrophic hormone, vasopressin and hormones involved in the reproductive system.

The following specific classes of therapeutic mammalian proteins, or above named proteins are specifically included for use with the invention: chemotactins; cytokines including interleukins 1,2 and RA (excluding interferon alpha); chemokines; enzymes including proteases and protease inhibitors; growth factors including acidic and basic fibroblast growth factors, epidermal growth factor, tumor necrosis factors, platelet derived growth factor, granulocyte macrophage colony stimulating factor, neurite growth factor and insulin-like growth factor-1, hormones including the gonadotrophins and somatomedians, immunoglobulins, lipid-binding proteins and soluble CD4.

Exemplary enzymes, as one of the therapeutic mammalian protein, or above named protein drug classes may also be packaged into the vesicles or micro-sponges of the invention for enhanced therapeutic efficacy. Individuals skilled in the art will recognize that the invention may benefit delivery of the following enzymes: urokinase, streptokinase, superoxide dismutase (SOD), catalase, phenylalanine ammonia lyase, L-asparaginase, pepsin, uricase, trypsin, chymotrypsin, elastase, carboxypeptidase, lactase, sucrase.

Specific therapeutic mammalian proteins, or above named proteins that are included are ciliary neurite transforming factor (CNTF), clotting factor VIII, erythropoietin, thrombopoietin, insulintropin, cholecystokinin, glucagon-like peptide I, intrinsic factor, Ob gene product, tissue plasminogen activator (tPA), brain-derived neurite factor. Administration of a therapeutic mammalian protein, or above named protein by the oral route is indicated where the subject suffers from a condition due to malabsorption of nutrients, e.g. deficiency in digestive enzymes, including lactase, intrinsic factor, sucrase, or transporters.

Where the target for protein therapy is the gastrointestinal tract, the entrapped therapeutic mammalian protein, or above named protein may be phenylalanine transporter (for phenylketonuria), lactase for lactase deficiency, intrinsic factor, or other brush border enzymes and transporters.

The therapeutic mammalian protein, or above named protein may be modified by posttranslational modification or applicable mutations of the gene coding for such protein or by synthetic attachment of carbohydrate groups to such protein.

The protein therapy concerned in this invention is aimed at therapy of mammalian subjects, be it bovine, canine, feline, equine, or human, or rodent subjects. Preferably the therapeutic mammalian protein, or above named protein used in the therapy is specific to man, i.e. obtained from recombinant production or chemical synthesis, but this requirement is not absolute, particularly if the amino acid sequence of the therapeutic mammalian protein, or above named proteins is highly conserved and non-immunogenic. Alternatively, the mammalian subject may have a condition which is amenable to treatment by a protein which is foreign to the mammalian subject, but may for instance enhance a normal metabolic process.

Exemplary diseases that are amenable to treatment using the subject invention, and exemplary, appropriate therapeutic mammalian protein, or above named proteins which can be used in treating these diseases, are discussed below.

The intestinal epithelium is the major absorptive surface in animals, and as such transports substances preferentially from the intestinal lumen into blood. It has been described in the literature that larger molecules may also be absorbed: in newborn animals immune responses are the result of the absorption of antibody proteins, various digestive enzymes from the pancreas, and other therapeutic mammalian protein, or above named proteins such as insulin, has been shown to cross the intestinal epithelium. Permeability to proteins has been seen primarily in the duodenum and terminal ileum, but proteins are also known to be absorbed from the lower portions of the large bowel, and suppositories have been used for this purpose therapeutically.

Proteins that are manufactured in the gut and targeted for secretion into the blood and are included in the ambit of the invention include hormones such as CCK (choleocystokinin), gastric inhibitory peptide (GIP), glucagon-like peptide I (GLPI), gut glucagon, islet amyloid polypeptide (IAPP), neuropeptide Y (NPY), polypeptide Y (PPY), secretin, vasoacitve intestinal peptide (VIP), and a variety of lipoproteins important in lipid metabolism.

Preferably, the use of active therapeutic mammalian proteins, or above named proteins, which may consist of multiple peptides are included in the invention. Similarly, therapeutic mammalian proteins, or above named proteins or peptides containing posttranslational modifications and processing that would normally occur in specific cells, but which may be absent in the cells targeted for treatment, are included. The use of modified forms of the therapeutic mammalian proteins, or above named proteins, where the modification carries a therapeutic advantage, are included in the invention. Such modifications may be aimed at characteristics such as protease resistance or enhanced activity relative to the wild-type protein.

### Assessment of protein therapy

The fatty acid based vehicles of the present invention can be used in connection with any therapeutic mammalian protein that is desired for administration. While the FA matrix can be used with therapeutic mammalian proteins, or above named proteins whose efficacy in intravenous therapies has not yet been tested, it can also be used with therapeutic mammalian proteins, or above named proteins of well-established efficacy (e.g. insulin, etc.). Furthermore, given the examples below, the ordinarily skilled artisan can readily determine that, since the FA matrix efficiently enhance absorption and efficacy of insulin in the bloodstream in an animal model after administration, then enhancement in the therapeutic effect of other therapeutic mammalian proteins, or above named proteins can also be readily achieved using the claimed fatty acid matrix of the invention.

Since the enhancement in efficacy of the claimed invention can be used in connection with a wide variety of therapeutic products, the therapeutic enhancement can be monitored in a variety of ways. Generally, such evaluation would be based on a comparative specific assay of a sample of blood from a subject treated with the same therapeutic molecule in similar concentrations in the presence and absence of the invention. Appropriate assays for detecting a therapeutic mammalian protein, or above named protein of interest in such samples are well known in the art. For example, a sample of blood can be tested for the presence of the therapeutic mammalian protein, or above named protein using an antibody which specifically binds the therapeutic mammalian protein, or above named protein in a RIA (radio immune assay). Such assays are performed quantitatively to determine the degree of enhancement. The assay may be enzyme-linked immunosorbent assay (ELISA), single-antibody radioimmuno-assay (RIA), double-antibody immunoradiometric assay (IRMA) or immunochemiluminometric assay. RIA techniques are usually less sensitive than IRMA and a typical working range is in the order of 0.5 - 200 mIU for IRMA. ELISA systems can increase the sensitivity 100 fold. The ELISA assay, as well as other immunological assays for detecting the therapeutic mammalian protein, or above named protein in a sample, are described in Antibodies: A Laboratory Manual (1988, Harlow and Lane, eds Cold Spring Harbor Laboratory, Cold Spring Harbor, N.Y.).

The amount of a specific therapeutic mammalian protein, or above named protein drug that traverse a biological barrier may be quantified by analytical methods such as high performance liquid chromatography, as exemplified below.

Alternatively, or in addition, the efficacy of the protein therapy can be assessed by measuring an activity associated with the therapeutic mammalian protein, or above named protein. Where the therapeutic mammalian protein, or above named protein is insulin, the efficacy of the therapy can be assessed by examining blood glucose levels of the mammalian subject or by measuring insulin (e.g., by using the human insulin radioimmunoassay kit, Linco Research Inc., St. Louis, Mo.).

### DESCRIPTION OF THE PREFERRED EMBODIMENTS

The invention will now be illustrated, purely by way of examples with reference to the following non-limiting description of Preparations and Examples. The invention concerns itself with the advantages it offers in the enhancement of efficacy of therapeutic mammalian proteins, or above named proteins by routes other than the parenteral route typically used in the administration of these drugs. In the following section some background on problems associated with and factors inherent in the intranasal, *peroral* and topical routes of administration of therapeutic mammalian proteins, or above named proteins are described.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, formulations and materials are now described.

### PREPARATION 1

### PREPARATION OF FORMULATIONS SUITABLE FOR USE AS A DELIVERY VEHICLE FOR USE IN DELIVERING A THERAPEUTIC MAMMALIAN PROTEIN, OR ABOVE NAMED PROTEIN TO MAMMALS

A formulation according to the invention may be made up as follows:
- Step 1:: A desired volume of water is saturated with nitrous oxide gas at ambient pressure using a pressure vessel and sparger. The vessel is connected to a supply of nitrous oxide via a flow control valve and pressure regulator. The closed vessel is supplied with nitrous oxide at a pressure of 2 bar for a period of 96 hours, it having been determined that at the aforementioned temperature the water is saturated with nitrous oxide over such period of time under the above-mentioned pressure. In the case of the preparation of the basic or stock formulation to be used as a carrier medium comprising a dispersion of vesicles unchlorinated water is used. The water is phosphate buffered to a pH of 5.8.
- Step 2:: The following fatty acid based compositions were made up: First, Vitamin F Ethyl Ester CLR 110 000 Sh.L. U./g obtained from CLR Chemicals Laboratorium Dr. Kurt Richter GmbH of Berlin, Germany which is composed mainly of 21% oleic acid, 34% linolenic acid, and 28% linoleic acid that are modified by esterification with an ethylene group of the carboxy terminal, was heated to 75°C. Secondly, pegylated, hydrogenated fatty acid, ricinoleic acid (also known by the INCI name as PEG-n-Hydrogenated Castor Oil), was heated to 80°C and mixed with the first group of fatty acid based Vitamin F Ethyl Ester at 70°C. The ratio of the first group of fatty acids to the latter fatty acid was generally 2.8:1.
- Step 3:: dl-α-Tocopherol of varying percentages (final concentration of between 0.1% when used as general anti-oxidant was added to the heated fatty acids mixture above.
- Step 4:: The buffered water was heated to 73°C and mixed with the fatty acid mix with the aid of a high speed shearer to a final concentration of between 3.2 and 4%, depending on the specific use of the preparation. This fatty acid mixture constituted the basic preparation that contains vesicles of sizes in the nanometer range as determined by particle size analysis on a Malvern sizer.
- Step 5:: To the basic preparation may be added additional ethylated fatty acids DHA (decahexonoic acid) and EPA (eicosapentanoic acid). The preferable amount of the two fatty acids for this invention was 0.5%. The addition of these fatty acids results in die formation of microsponges rather than vesicles, with particles between 2-5 µm in size, as determined by particle size analysis on a Malvern sizer.

### EXAMPLE 1

### THE ENHANCEMENT IN INSULIN PLASMA LEVELS AND INSULIN EFFICACY BY ITS ENTRAPMENT IN THE FA-BASED PARTICLES OF THE INVENTION

### Animal studies

Male Sprague Dawley rats with a body mass of between 240 and 336g were used as experimental *in vivo* model to investigate the absoption and efficacy enhancing capabilities of the current invention. Besides other advantages of this animal as model, the anatomical sequence and morphology of the animal's gastro-intestninal and nasal physiology and biochemistry show several similarities to that of the human.

In this study, insulin was directly administerd into the stomach, ileum or duodenum of the animals. The experimental procedures of the *in vivo* method are well doumented in the literature. Six animals were used for each group in the study. Rats were fasted 18 hours prior to drug administration but water was supplied *ad libitum.* The rats were kept under artificial conditions to create the ideal environment for the optimum growth and health of the animals. Infection with pathogen organisms was also minimised and variables were kept constant. The conditions under which the rats were bred and kept were 21°C with a relative humidity of 55%, a light intensity of between 350-400 lux one meter above floor level with light cycles of 12 hours light and 12 dark and 18 air changes per minute.

The study has a parallel design: the experimental animals were arranged in various test groups according to the different treatments and each animal received a single treatment. The control group received a single dose of insulin in saline solution to determine the absorption without the presence of any enhancing agent. Subcutaneous injection of insulin in one group was used to validate assaying procedures. The normal rat glucose and insulin profile was determined in a group that received only saline; this group acted as biological reference.

Recombinant human insulin was obtained from Sigma-Aldrich (South Africa). Insulin was entrapped in the FA-based vesicles or microsponges preparations of the invention, prepared as described above, according to the concentration and volume of formulation required. Before entrapment the FA-based preparations were heated to 31°C in a waterbath. After addition of the insulin these formulations were shaken gently for 30 minutes to allow the insulin to be entrapped in the FA preparation. After entrapment the formulations were kept at 4°C until administration. A dose of 50IU/kg was administered in each of the test and control groups, except for the insulin that was administered intravenously, the dose of which was 0.5IU/kg per animal, while that for subcutaneous administration was 4IU/kg.

Cannulation of the *artery carotis communis,* ensured that sufficient blood volumes from the same rat at different time intervals could be obtained for analysis. Anaesthesia was induced by halothane and lasted for ± 3 hours. All surgical procedures necessary for the cannulation of the *carotis communis* were carried out while the rats were under anaesthesia. A sterile PVC cannula (Fine Bore Polythene tubing, 0.58 mm ID (0.96 mm OD) REF 800/100/200/100, UK) which was filled with a saline-heparin solution at body temperature and connected to a syringe, was guided into the artery. A 5.0% heparin solution was used to avoid blood clotting in the cannula. Anaesthesia was induced in each rat by their inhalation of a concentration of 4.0% v/v liquid halothane (Fluothane^{®}, Zebeca SA (Pty) Ltd, Woodmead, RSA) in a closed glass container. The rats were removed from the container upon loss of conciousness. Anaesthesia was maintained by alternate use of 2.0 and 4.0% halothane and medical oxygen. A constant body temperature of 37 °C was maintained by placing each rat on a small thermal electric blanket for the duration of the experiment. At the end of each experiment euthanasia was performed, before the rat regained conciousness, by deepening the anaesthesia with the 4.0% halothane until breathing ceased.

Abdominal surgical procedures were performed under the same conditions. The skin of the ventral abdomen was shaved and disinfected. A midline abdominal incision (laparotomy) of approximately 2 cm was made through the linea alba caudal to the sterno without cutting the intestines. Either the stomach, ileum or colon was identified and lifted out of the incision for administration of the insulin formulations where after it was replaced in the abdominal cavity in its correct anatomical position. The incision was covered with sterile gauze and kept moist with a saline-heparin solution to prevent dehydration.

The relevant formulation was injected directly into the specific area. Intra-gastric injections were made into the lumen of the stomach, after which the stomach was ligated at the start of the duodenum to ensure that the formulations were not transported to the small intestine by peristaltic movement. Intra-ileal administrations were made directly into the lumen of the small intestine, 7 cm from the stomach exit into the intestines. The small intestine was not ligated to ensure normal absorption of insulin with normal transit times. Intra-colonic administrations were made directly into the colon. The colon was ligated at the proximal end to ensure that formulations did not pass back into the ileum. All administrations were done gently and slowly to prevent any spillage.

In the case of the groups receiving intravenous injections, a single administration with a volume of 200µl/body weight containing 0.5IU/kg was made into the tail of each rat. The efficacy of recombinant human insulin in rats was hereby confirmed and the relative bioavailability of the test formulations could be calculated with this group as reference. Subcutaneous injections were made just beneath the abdominal skin in volumes of 300µl/250g of body weight, resulting in a dose of 4IU/kg, which seemed comparable with that of commercial preparations.

Blood samples consisting of one ml of blood were collected at 0, 5, 10, 15, 30, 60,120 and 180 minutes after drug administration for the determination of blood glucose values and insulin plasma levels. Blood glucose levels are a reflection of the therapeutic efficacy of each formulation. Blood glucose levels were measure with a Glucometer® II reflectance meter. A single drop of blood was applied to a Glucostix®reagent strip (Bayer, South Africa), blotted after 30 seconds and the glucose in mmol/l was obtained after 20 seconds.

Plasma insulin levels of the plasma samples were determined by the quantitative measurement of human insulin in the collected plasma using a human specific radioimmunoassay (RIA) kit obtained from LINCO Research, USA. The specificity of the the human insulin specific RIA was stated to be 100% for human insulin and 0.1 % for rat insulin, with no cross-reactivity with human pro-insulin. The limit of detection of the kit was 2µIU/ml.

### Results

### Intra-gastric administrations

The enhancement in the absorption of insulin by entrapment in the particles of the invention is illustrated in Table 1. Table 1 shows the average plasma levels found for two experimental groups of animals consisting of 6 rats each, and each of which have received a single administration of 50IU/kg insulin in the indicated form. The times at which the blood samples were collected are indicated.

**Table 1: Intra-gastric plasma levels**

| Time | Insulin in saline (µlU/ml) | Insulin in FA vesicles (µlU/ml) |
|---|---|---|
| 0 | 12.95 | 8.28 |
| 5 | 19.09 | 49.37 |
| 10 | 15.29 | 18.85 |
| 15 | 11.1 | 19.13 |
| 30 | 13.09 | 19.57 |
| 60 | 11.8 | 16.06 |
| 120 | 10.48 | 26.48 |
| 180 | 13.51 | 32.56 |
| Parameter | Insulin/saline | FA vesicles |
| Cₘₐₓ(µlIU/ml) | 19.09 | 49.37 |
| Tₘₐₓ (minutes) | 5 minutes | 5 minutes |
| AUC | 2175 | 4282 |
| Enhancement in AUC | | 96.873563% |
| Enhancement in Cₘₐₓ | | 158.62% |

The vesicles of the invention thus aided the absorption of insulin and maintained a higher concentration of insulin in the blood through the course of the 3-hour experiment. The initial absorption is reflected by the Tₘₐₓ at 5 minutes for both groups, but the results seem to indicated that insulin is protected in the blood against degradation by entrapment into the vesicles and gradually released, as levels appear to still be rising after 3 hours, whereas in the absence of vesicles, insulin levels seem to be at base level. As a result of the still rising trend, the absolute bioavailability as found after intravenous administration of insulin, cannot be accurately calculated but when corrected for dosage the absolute bioavailability is at least doubled by entrapment into the vesicles of the invention. The increase in relative bioavailability and therapeutic levels by the vesicles of the invention are indicated by the enhancement in AUC and Cₘₐₓ respectively.

The enhancement in therapeutic efficacy was measured by determining the effect of the various administrations on the blood glucose levels.

| Table 2: Decrease in the % of blood glucose levels | | |
|---|---|---|
| Parameter | Insulin in saline | Insulin in FA vesicles |
| Tₘₐₓ (minutes) | 30 | 15 |
| Cₘₐₓ (µlU/ml) | 4.1 | 15.3 |
| AUC | 275.5 | 613.5 |
| Enhancement AUC | | 122.686% |
| Enhancement Cₘₐₓ | | 273.1707% |

The results portrayed in Table 2 confirm that the enhancement in the therapeutic efficacy by the invention is larger than that in the relative absorption/bioavailability (compare enhancement of AUC= 96.87.62% in Table 1 and 122.68% in Table 2). This supports the hypothesis that entrapment of insulin in the vesicles is protecting the insulin from proteolytic degradation in the plasma.

### Intra-ileal administrations

Results obtained from blood plasma insulin levels after intra-ileal administration of insulin in 0.9% saline and entrapped in the vesicles of the invention are reflected in table 3. These results indicate that the ileum presents with ideal characteristics for optimum insulin absorption. Compared to the the ileum, the stomach did not provide as much insulin absorption. A vast increase of blood plasma insulin levels of up to 243.8µIU/ml is reached after 5 minutes with vesicle-entrapped insulin, compared to the 39.3µIU/ml of the same dose in saline.

| **Table 3: Intra-ileal plasma levels** | | |
|---|---|---|
| Time | Insulin in saline (µlU/ml) | Insulin in FA vesicle (µlU/ml) |
| 0 | 20.83 | 7.22 |
| 5 | 39.3 | 243.8 |
| 10 | 33.16 | 172.58 |
| 15 | 15.49 | 47.605 |
| 30 | 19.86 | 35.0775 |
| 60 | 19.49 | 26.084 |
| 120 | 14.9 | 26.236 |
| 180 | 13.88 | 25.7675 |
| Parameter | Insulin in saline | Insulin in FA vesicles |
| AUC | 569.1 | 4178 |
| Tₘₐₓ | 5 | 5 |
| Cₘₐₓ | 37.73 | 242.8 |
| % enhancement AUC | | 634.1416% |
| % enhancement Cₘₐₓ | | 541.3994% |

Table 4 shows that the enhanced therapeutic efficacy observed for vesicle-entrapped insulin after intra-gastric administration is also present after intra-ileal administration. Again the results confirm that the enhancement in the therapeutic efficacy by the invention is larger than that in the relative absorption/bioavailability (compare enhancement of AUC= 634.1416% in Table 3 and 42774.73% in Table 4). The protection of insulin from proteolytic degradation by entrapment of insulin in the vesicles in the plasma is clear.

| Table 4: Decrease in the % of blood glucose levels | | |
|---|---|---|
| Parameter | Insulin in saline | Insulin in FA vesicles |
| Tₘₐₓ (minutes) | 10 | 10 |
| Cₘₐₓ (µIU/ml) | 4.0 | 42.3 |
| AUC | 13.81 | 5921 |
| Enhancement AUC | | 42774.73% |
| Enhancement Cₘₐₓ | | 957.50% |

The enhancement in therapeutic efficacy by entrapment in the vesicles of the invention is 437.7 times. A comparison between the AUC's observed after intravenous insulin administration and intra-ileal administration can be used to determine absolute therapeutic efficacy. The absolute therapeutic efficacy of the insulin entrapped in vesicles was found to be 0.69 times of that observed after IV administration and 0.72 times that of subcutaneously administered insulin. Whilst the therapeutic efficacy of the vesicle-entrapped insulin is still somewhat lower than that of parenteral administrations, no glucagon response was observed for this therapy over the period of the study, with the % blood glucose levels staying under 100%, whilst a glucagon or hyperglycaemic response was observed with both the parenteral administration routes.

### Intracolonic administration

Entrapment of insulin in vesicles led to enhanced insulin plasma levels (1.63 times) and therapeutic response (7.8 times) when compared with the insulin in saline administration.

### Conclusion

The entrapment of insulin into the vesicles of invention gave a near ideal insulin response, resulting in sufficient therapeutic efficacy but no hyperglyceamia.

### EXAMPLE 2

### COMPARATIVE NASAL ADMINISTRATION OF INSULIN

In this Example, insulin, as described in Example 1, was administered nasally, using the same procedures for the induction and maintenance of aneasthesia as described for Example 1. The cannulation of the *carotis communis* artery for the collection of blood samples was also performed as described in Example 1 as was the determination of plasma levels and blood glucose levels.

### Results

In table 5 the observed plasma levels after nasal administration of insulin at a dosage of 8 and 12IU/kg body weight are presented.

| Table 5: Comparative plasma insulin levels after intranasal administration | | | | | | |
|---|---|---|---|---|---|---|
| Time | Insulin in saline | | Insulin in FA vesicles | | Insulin in FA microsponges | |
| | 8lU/kg | 12lU/kg | 8lU/kg | 12lU/kg | 8lU/kg | 12lU/kg |
| 0 | 0.654 | 2.278 | 0.725 | 0.162 | 1.738 | 1.275 |
| 5 | 1.996 | 2.348 | 10.893 | 6.074 | 10.893 | 8.195 |
| 10 | 1.101 | 0.758 | 35.718 | 52.935 | 39.05 | 39.237 |
| 15 | 3.6425 | 1.278 | 47.358 | 68.785 | 64.192 | 54.547 |
| 30 | 6.734 | 1.997 | 44.036 | 67.775 | 73.15 | 61.21 |
| 60 | 4.142 | 2.593 | 37.48 | 55.096 | 61.758 | 49.957 |
| 120 | 2.665 | 3.82 | 128.576 | 44.01 | 129.33 | 150.015 |
| 180 | 3.838 | 17.452 | 302.478 | 59.63 | 220.205 | 154.762 |
| AUC | 666.5 | 948.4 | 20175 | 9417 | 19687 | 18055 |
| Tₘₐₓ | 30 | 180 | 180 | 15 | 180 | 180 |
| Cₘₐₓ | 6.734 | 17.45 | 302.5 | 68.79 | 220.2 | 154.8 |
| AUC enhancement | | | 29.27 | 8.93 | 28.54 | 18.04 |
| Cₘₐₓ enhancement | | | 43.92 | 2.94 | 31.70 | 7.87 |

The plasma insulin levels are increased by the vesicles and microsponges of the invention after intranasal administration of 8IU/kg body weight, as reflected by the AUCs, is dramatic, with a 29.27 times enhancement in the case of the vesicles and 28.54 times in the case of the microsponges. It would thus seem that at this dosage, the particles of the invention enhanced the absorption and transport of insulin into the plasma equally. The enhancements found for the higher dosage (12IU/kg body weight) differs significantly for the two types of particles (8.9 times for the vesicles and 18.04 times for the sponges). The difference may be explained by the fact that in the case of the lower dosage administered by vesicles, the insulin plasma level is still increasing, indicating that the transport to or release into the plasma is slower. In fact, in all the groups that received insulin by way of the particles of the invention, the real enhancement is higher than that portrayed, as in none of these cases has the blood drug profiles returned to base level.

| Table 6: The extent of the % blood glucose decrease after intranasal administration | | | | | | |
|---|---|---|---|---|---|---|
| Time | Insulin in saline | | Insulin in FA vesicles | | Insulin in FA microsponges | |
| | 8lU/kg | 12lU/kg | 8lU/kg | 12lU/kg | 8lU/kg | 12lU/kg |
| 0 | 0 | 0 | 0 | 0 | 0 | 0 |
| 5 | -1.18 | 6.48 | 12.35 | 8.16 | 7.27 | 3.4 |
| 10 | 5.58 | -0.08 | 11.72 | 5.65 | 19.17 | 13.18 |
| 15 | 10.033 | -4.4 | 18.22 | 6.3 | 19.22 | 8.67 |
| 30 | 5.27 | -8.32 | 12.68 | 10.32 | 36.47 | 13.87 |
| 60 | 3.47 | -13.98 | 16.52 | 19.8 | 34.53 | 30.32 |
| 120 | 2.05 | -30.7 | 27.67 | 39.525 | 36.95 | 35.26 |
| 180 | -26.58 | -40.55 | 44.38 | 38.2 | 54.94 | 72.375 |
| AUC | 46 | 32.2 | 4323 | 4773 | 6564 | 6133 |
| Tₘₐₓ | 15 | 5 | 180 | 120 | 180 | 180 |
| Cₘₐₓ | 10.03 | 6.48 | 44.38 | 39.53 | 54.94 | 72.38 |
| AUC enhancement | | | 92.98 | 147.23 | 141.70 | 189.47 |

The results in table 6 reflect the therapeutic efficacy of the administered insulin. These results show that the enhancement in therapeutic efficacy by the particles of the invention is again larger than the enhancement in the drug plasma levels, with the therapeutic efficacies enhanced by 92.98 times and 141.7 times for the vesicle and microsponges at a dosage of 8IU/kg body weight respectively and enhancements after administration of 12IU/kg body weight of 147.23 and 189.47 times for vesicles and microsponges respectively. From this table it is also clear that the microsponges increased the therapeutic efficacy more than the vesicles, despite the lower plasma levels observed (see table 5). As the same samples were used in the determination of the plasma levels and the blood glucose levels, the result is not due to sample- or inter-animal variation. The explanation for this discrepancy is probably that entrapped insulin may not be recognized by the antibodies of the RIA before the insulin is released, but it may still exert its therapeutic effect.

### EXAMPLE 3

### TRANSDERMAL DELIVERY OF ARGININE VASOPRESSIN WITH FA-BASED VESICLES OF THE INVENTION

The stratum corneum is known to be a nearly impenetrable barrier, resulting in a considerable amount of resistance against percutaneous absorption of most substances. Protein or pharmaceuticals generally illustrate poor penetrability due to their large molecular sizes and relatively hydrophilic nature.

In order to test the feasibility of transdermal delivery of macromolecules, the peptide hormone arginine vasopressin (AVP) (MW = 1084.23 Da) was used as a model compound. AVP is regarded as a relative 'small' macromolecule and represents peptides in the molecular weight range of 1000-1500 Da. It is an endogenous neurohypophyseal, nonapeptide hormone and is commonly utilised in the diagnosis and therapy of diabetes insipidus and nocturnal enuresis in the synthetic form of ℓ-deamino-8-D-arginine-vasopressin (DDAVP or desmopressin).

Previous studies on the transdermal absorption and/or delivery of arginine vasopressin used iontophoresis at low currents and chemical enhancers in low quantities in tandem to circumvent any potential adverse reactions, toxicity and irreversible structural changes. Three of these studies were conducted using rat skin. The remainder of the studies involved investigations into the electrical parameters and physicochemical considerations of AVP transdermal permeation, with references to earlier studies. Other transdermal studies that involved AVP as the model peptide focused on the effects of buffer pH and concentration, as well as proteolytic enzyme inhibitors, on the stability of AVP and its degradation in rat and human cadaver skin. Bestatin is a potent, competitive and specific aminopeptidase inhibitor with an affinity for leucine aminopeptidase (LAP), aminopeptidase B (APB) and tri-aminopeptidase. Bestatin has been shown to exhibit antitumor as well as antimicrobial activity, but is also known to act as an immune response modifier and analgesic by enkephalinase inhibition. Bestatin hydrochloride was used in the present study to selectively inhibit aminopeptidases present inside and on the surface of the skin, which could potentially degrade the studied active.

Hepes (4-(2-Hydroxyethyl)piperazine-1-ethanesulfonic acid) at pH 5.5 was used as the receptor phase in the transdermal diffusion studies and as the solvent for all solutions prepared. It was also utilised as the aqueous phase in the manufacturing of the particles of the present invention. In this study the comparative *in vitro* transdermal transport of AVP across human skin was investigated.

### Study procedures

Abdominal skin was obtained from Caucasian female patients after cosmetic surgery. The full-thickness skin was frozen at -20 °C not longer than 24 hours after removal. Before preparation, the skin was thawed at room temperature, remaining blood wiped off with tissue paper and all excess adipose tissue carefully removed with a scalpel. Epidermal layers were separated by immersing the skin for 1 min in water at 60 °C. The epidermal layer was gently flayed from the underlying tissue with a forceps. Special care was taken to ensure that the integrity of the stratum corneum remained intact. The skin sections were floated on top of Whatman® filter paper after ensuring that the stratum corneum side of the epidermal skin faced upwards. The skin sections were then left to air dry. The prepared skin samples were wrapped in aluminium foil and sealed in plastic bags. The samples were kept frozen at -20 °C until used. Before a diffusion study was conducted the frozen pieces of skin were thawed at room temperature and examined for defects. The skin was cut into circles with a diameter of ± 10 mm before mounting them in the diffusion apparatus.

The epidermal layer was mounted on the lower half of vertical Franz diffusion cells (PermeGear Inc., Bethlehem, PA, USA) with a receptor capacity of approximately 2 ml and a 1,075 cm² diffusion area with the stratum corneum facing the donor half-cell. For each individual diffusion study a single source of skin was employed to minimise variation between skin samples. Stirring of the contents of each receptor phase was continued throughout the entire experiment, using a small magnetic stirring bar. The donor compartment was placed on the lower half, with the skin acting as a seal between the two halves, sealed with vacuum grease and fastened together with a metal clamp. After filling both compartments of the diffusion cells with physiological saline, cells were equilibrated for 1 hour in a water bath held at a constant temperature of 37±0.5 °C giving a membrane temperature of 32±1.0 °C.

The integrity of the skin was ascertained with the aid of a Model 6401 LCR Databridge (H. Tinsley, Inc., Croydon, Surrey, UK) set in the resistance mode (R), in the parallel equivalent circuit mode (PAR) and with an alternating current (AC) frequency of 1000 hertz (Hz) (Fasano et al., 2004). Impedance measurements were taken in the donor and receptor compartments simultaneously as an indication of the relative integrity of the skin sample. These impedance measurements were repeated after completion of the diffusion study. Physiological saline was used in the integrity assessments instead of Hepes buffer as Hepes buffer ions have much lower mobility through skin during iontophoresis as compared to the major counter ion chloride. It was therefore hypothesised that if Hepes was unable to transfer a charge during iontophoresis it would probably not be able to do so during impedance measurements. This hypothesis was confirmed during pilot studies (data not shown) where impedance measurements, with Hepes buffer as the donor and receptor phases, were attempted. The compartments were emptied after conclusion of the impedance measurements.

The receptor phase, Hepes buffer (0.025 mM, pH = 5.5±0.5), was sonicated for 15 min to remove air bubbles and avoid the build up of air pockets and heated to 37 °C. The receptor compartment was filled with the buffer before adding the drug-containing solution to the donor compartments. Care was taken to ensure that no air bubbles were trapped in the receptor compartment or underneath the skin. To initiate an experiment, the donor compartment of each cell was charged with 1000 µl (1 ml) of either an aqueous solution of the active in Hepes buffer or the drug entrapped in the FA vesicles, depending on the experiment, and immediately covered with Parafilm® to prevent any liquid from evaporating. At predetermined intervals (0.5; 1; 1.5; 2; 4; 6; 8; 10; and 12 hours), the entire content of the receptor compartment was withdrawn, and replaced with fresh 37 °C Hepes buffer to ensure that sink conditions existed throughout the experiment. One hundred microlitres (100 µl) of each sample was directly assayed by high-performance liquid chromatography (HPLC) to determine the drug concentration in the receptor fluid.

[⁸Arg]vasopressin (AVP) (acetate salt, MW = 1084.23) was entrapped in the vesicles of the present invention at a concentration of 150 µg/ml for approximately 30 minutes at room temperature and kept in a fridge at 2-8 °C for 24 hours before commencement of an experiment. Entrapment of the peptide in the vesicles was confirmed with the aid of confocal laser scanning microscopy (CLSM) on a Nikon PCM 2000 CLSM, using a medium (10µm) pinhole and a 60x, 1.4D ApoPlanar oil immersion objective. The microscope was equipped with a krypton laser (wavelengths: excitation 488 nm, emission 515 nm) and a helium/neon laser (wavelengths: excitation 505 nm, emission 564 nm). For this purpose the AVP was labeled with the reactive dye Alexa Fluor® 430 and the particles of the invention with Nile red according to the instructions of the manufacturer (Invitrogen, Leiden, Netherlands). Alexa Fluor® 430 has fluorescence with a maximum emission of photons at 540 nm while Nile red labeled particles had an emission wavelength of between 640 and 650 nm. The entrapment efficiency could thus be monitored. The reference solution contained 150 µg/ml AVP dissolved in Hepes buffer at a concentration of 25mM. When included, the concentration of bestatin hydrochloride in both the reference and test formulations was 300 µg/ml.

To determine the amount of AVP transported across the skin epidermis, high-performance liquid chromatography (HPLC) analyses of AVP found in the receptor phases were performed. An Agilent 1100 series HPLC equipped with a gradient pump, autosampler and diode array UV detector was interfaced with Chemstation Rev. A.08.03 data acquisition and analysis software. A reversed phase chromatography column (Macherey-Nagel LiChrospher® 100 RP18 ec column; 4 mm x 250 mm, 5 µm particle size, pore size 100 A, endcapped), a mobile phase of 100% acetonitrile (ACN) and an aqueous phase of 0.1% trifluoroacetic acid (TFA) in HPLC grade water was used. Injection volume was set at a default value of 100µl. The following gradient elution was used: 5 % ACN up until 2 minutes, then a linear increase in ACN to reach 80 % after a further 8 minutes. Stop time was at 10 minutes and a 4-minute post time allowed the instrument to return to the initial ACN concentration. The preservation time of AVP was approximately 7.3-7.5 minutes and that of bestatin approximately 8.2-8.5 minutes. The flow rate was kept constant at 1 ml/min and analyses were performed at ambient room temperature (25±1 °C). The DA detector was used to detect the absorbance of the effluents at a wavelength of 210 nm.

The cumulative amount of AVP permeated per unit time skin area was plotted against time. With the possible exception of the passive flux, the plots exhibited biphasic character, thus the slopes of the linear portions of the plots between zero and two hours, as well as two and twelve hours, were estimated as the steady-state fluxes for the two time periods. The yield of each cell was depicted as a percentage of the applied concentration and based on these values, data of cells with yield values of 2 % and less for arginine vasopressin and values of 20 % and less for bestatin were selected for inclusion in the dataset. All the results were expressed as mean ± S.D.

### Results and discussion

CLSM analysis confirmed entrapment of arginine vasopressin within the vesicles of the invention. The *in vitro* permeation of AVP with the aid of the FA-based vesicles was investigated in the absence and presence of the aminopeptidase inhibitor bestatin. It was also compared to the control (permeation of AVP in combination with bestatin in Hepes buffer) and the passive flux (AVP in Hepes buffer). The average *in vitro* permeation profiles of AVP under the different circumstances are shown Table 7. The fluxes of each of the groups assayed were obtained from at least 6 diffusion cells and only means are portrayed. For example, AVP in FA vesicles in table 7 represents the mean flux determine from 18 cells, AVP (+bestatin) in Hepes buffer group from 6 cells and AVP (+bestatin) in FA vesicles group from 21 cells.

The transport of AVP across the prepared skin exhibited a biphasic character, with the first phase from time zero to two hours, and the second from time two to twelve hours. The fluxes of the transport for the different phases can be seen in Table 7. Flux was calculated for two different periods of time: t = 0 - 2 hours and t = 2 - 12 hours. The majority of AVP flux seemed to take place during the first two hours of diffusion.

| **Table 7: Fluxes for AVP in buffer and entrapped in FA vesicles plus/minus bestatin.** | | | |
|---|---|---|---|
| Donor phase | Flux (µg/ml/h): 0 - 2 hours | Flux (µg/ml/h): 2 - 12 hours | Total flux |
| AVP in Hepes buffer | 0.0374 | 0.0175 | 0.0549 |
| AVP in FA vesicles | 0.1495 | 0.0376 | 0.1871 |
| AVP (+bestatin) in FA vesicles | 0.2182 | 0.0575 | 0.2692 |

The vesicles of the present invention significantly increased the flux of AVP when compared to the observed passive flux. With the inclusion of bestatin, an even more distinct increase in the flux of AVP was observed. In the case of the exclusion of bestatin, the AVP flux approaches steady-state, indicating a decline in AVP permeation. The biphasic character of the flux may be ascribed to gradual depletion of the AVP after two hours or, in the case of the presence of bestatin, the depletion of bestatin and the consequential decline in AVP flux. It is also possible that the proteolytic enzymes, aminopeptidase and trypsin, might diffuse through the skin concomitantly with the AVP and degrade the active while in the receptor phase.

The results thus indicate that entrapment of AVP in the vesicles of the invention is capable of enhancing *in vitro* delivery of a peptide into the skin at least 2.4 times.

### Advantages and potential application of the invention

An important advantage of the present invention is that it allows administration of therapeutic mammalian protein, or above named protein drugs by administration routes other than by injection. Despite the conventional wisdom that any protein in the gastrointestinal tract would be destroyed rapidly by the digestive process (either by stomach acid or intestinal enzymes), or that the molecular sizes of these drugs are too large for nasal or topical delivery, entrapment of therapeutic mammalian proteins, or above named proteins into a fatty acid matrix, followed by intracellular release of the therapeutic mammalian proteins, or above named proteins from such matrices have been shown to be successful in the present invention. Using this invention therapeutic mammalian protein, or above named protein drugs are shown to be
(a) absorbed better into the circulation as confirmed by its quantification in the plasma of the animals, and
(b) undegraded as confirmed by means of antibody assays and
(c) effective as indicated by its enhancement of therapeutic effect.

The flexibility of this technology allows for the absorption, distribution and delivery of a wide variety of therapeutic mammalian protein, or above named protein pharmaceuticals, systemically as well as locally, making it well suited for a broad spectrum of therapeutic applications. The FA-based particles can be manipulated in terms of their structure, size, morphology and function, depending on the type and size of the drug molecules to be delivered, the therapeutic indication and the required circulating half life of the drug.

Yet another advantage of the invention is the possibility of increasing the circulating half life of the therapeutic mammalian protein, or above named protein drug. This increase may be the result of:
(a) an inhibition of enzymatic degradation,
(b) a decrease in therapeutic mammalian protein, or above named protein recognition of immune cells, leading to an immunogenic response - no humoral immune responses against the invention itself were found after either oral, nasal, topical or subcutaneous administration.;
(c) stabilization of the stereochemistry of the therapeutic mammalian protein, or above named protein by entrapment into the FA matrix of the invention.

The above then also indicates another advantage of the invention: that potential deleterious side-effects due to immunogenic responses are minimized by masking of therapeutic mammalian protein.

One of the most prominent advantages of the invention is the use of essential and other therapeutic fatty acids in the composition of the invention. It is well known from the literature that these fatty acids contribute *inter alia* to the maintenance of cell membrane integrity, modulation of the immune system, energy homeostasis, and the antioxidant status of the cell. The FA component contributes to the transport of the particles of the invention and their entrapped drugs across the cell membrane. These characteristics of the FA-based particles affords it significant advantages over other delivery systems.

In terms of the various administration routes, the ability to use the invention for nasal delivery is yet another advantage. Nasally administered drugs have to be transported over a very small distance before absorption, in comparison to orally administered drugs. Nasally administered drugs are not exposed to extremely low pH values or degrading enzymes; the first pass metabolism is also eliminated by this route. Drugs for nasal administration can be formulated as fatty acid based drops or even sprays. Various factors synergistically enhance the permeation of nasally administered drugs: the nasal cavity offers a highly vascularized epithelium, a porous endothelial membrane and a relatively large surface area due to the presence of a large number of microvilli. Fatty acid-based gels may be a viable option when longer lasting drug release is required.

In a similar fashion, the invention holds promise for topical administration. The administration of drugs through the skin has many advantages such as the elimination of first pass metabolism by the liver, no gastrointestinal effects or degradation and it is not invasive. The FA-based nitrous oxide saturated particles can be incorporated in creams, lotions, ointments and patches which makes it extremely versatile and suitable for both membrane and drug reservoirs in transdermal patches. As shown in Example 3, the FA particles are able to penetrate human skin, which means that it is possible to administer active compounds via the topical route.

These and other objects, advantages and features of the present invention will become apparent to those persons skilled in the art upon reading the details of the formulations and methodology herein described.

## Claims

1. A formulation for use for the administration of at least one therapeutic mammalian protein to a mammal, and for enhancing the absorption, distribution and release of the at least one therapeutic mammalian protein in or on the mammal, the formulation consisting of at least one therapeutic mammalian protein in a micro-emulsion which micro-emulsion is constituted by a dispersion of vesicles or microsponges of a fatty acid based component in an aqueous or other pharmacologically acceptable carrier in which nitrous oxide is dissolved, the fatty acid based component comprising at least one long chain fatty acid based substance selected from the group consisting of free fatty acids and derivatives of free fatty acids, which derivatives thereof are selected from the group consisting of the C₁ to C₆ alkyl esters thereof, the glycerol-polyethylene glycol esters thereof, and the reaction product of hydrogenated and unhydrogenated natural oils composed largely of ricinoleic acid based oils with ethylene oxide.

2. A formulation for use according to claim 1 for the administration to a mammal of at least one protein wherein the at least one protein is selected from the group consisting of insulin, parathyroid hormone, parathyroid-like hormone, glucagon, insulinotrophic hormone, vasopressin and hormones involved in the reproductive system, chemotactins; cytokines including interleukins 1,2 and RA but excluding the interferons; chemokines; enzymes including proteases and protease inhibitors; growth factors including acidic and basic fibroblast growth factors, epidermal growth factor, tumor necrosis factors, platelet derived growth factor, granulocyte macrophage colony stimulating factor, neurite growth factor and insulin-like growth factor-1, hormones including the gonadotrophins and somatomedians, immunoglobulins, lipid-binding proteins and soluble CD4, urokinase, streptokinase, superoxide dismutase (SOD), catalase, phenylalanine ammonia lyase, L-asparaginase, pepsin, uricase, trypsin, chymotrypsin, elastase, carboxypeptidase, lactase, sucrase, ciliary neurite transforming factor (CNTF), clotting factor VIII, erythropoietin, thrombopoietin, insulintropin, cholecystokinin, glucagon-like peptide I, intrinsic factor, Ob gene product, tissue plasminogen activator (tPA), brain-derived neurite factor, phenylalanine transporter, brush border enzymes and transporters, and for enhancing the absorption, distribution and release of the at least one protein in or on the mammal, the formulation consisting of the at least one protein in a micro-emulsion which micro-emulsion is constituted by a dispersion of vesicles or microsponges of a fatty acid based component in an aqueous or other pharmacologically acceptable carrier in which nitrous oxide is dissolved, the fatty acid based component comprising at least one long chain fatty acid based substance selected from the group consisting of free fatty acids and derivatives of free fatty acids.

3. The formulation of claim 1 wherein the composition also contains the antioxidant dl-α-tocopherol or a stable derivative thereof.

4. The formulation of claim 1 wherein the composition includes a protease inhibitor.

5. The formulation of claim 1 wherein the dispersion is **characterized in that** at least 50% of the vesicles are of a diametrical size of between 80 nanometer and 3µm and that of the microsponges between 1.5 and 6.0µm.

6. The formulation of claim 1 or 2 wherein the fatty acid based component is selected from the group consisting of oleic acid, linoleic acid, alpha-linolenic acid, gamma-linolenic acid, arachidonic acid, eicosapentaenoic acid [C20:5ω3], decosahexaenoic acid [C22:6ω3], and ricinoleic acid, and derivatives thereof selected from the group consisting of the C₁ to C₆ alkyl esters thereof, the glycerol-polyethylene glycol esters thereof, and the reaction product of hydrogenated and unhydrogenated natural oils composed largely of ricinoleic acid based oils with ethylene oxide.

7. The formulation of claim 1 wherein the fatty acid component of the micro-emulsion is constituted by the mixture of esterified fatty acids known as Vitamin F Ethyl Ester.

8. The formulation of claim 1 wherein the dispersion is in the form of microsponges and is constituted by very long chain polyunsaturated fatty acids selected from eicosapentaenoic acid [C20:5ω3] and decosahexaenoic acid [C22:6ω3] or a mixture of both.

9. The formulation of claim 8 wherein the fatty acid component further includes the reaction product of hydrogenated natural oils composed largely of ricinoleic acid based oils with ethylene oxide or modified derivatives thereof.

10. The formulation of claim 1 wherein the protein is insulin.

## Patentansprüche

1. Formulierung zur Verwendung für die Verabreichung von wenigstens einem therapeutischen Säugetierprotein an ein Säugetier und zum Verstärken von Absorption, Verteilung und Freisetzung des wenigstens einen therapeutischen Säugetierproteins in oder an dem Säugetier, wobei die Formulierung aus wenigstens einem therapeutischen Säugetierprotein in einer Mikroemulsion besteht, wobei die Mikroemulsion von einer Dispersion von Vesikeln oder Mikroschwämmen einer Komponente auf Fettsäurebasis in einem wässrigen oder anderen pharmakologisch akzeptablen Träger gebildet wird, in dem Distickoxid aufgelöst ist, wobei die Komponente auf Fettsäurebasis wenigstens eine Substanz auf der Basis einer langkettigen Fettsäure umfasst, ausgewählt aus der Gruppe bestehend aus freien Fettsäuren und Derivaten von freien Fettsäuren, wobei die Derivate davon ausgewählt sind aus der Gruppe bestehend aus den C₁ bis C₆ Alkylestern davon, den Glycerol-Polyethylen-Glykolestern davon und dem Reaktionsprodukt von hydrierten und unhydrierten natürlichen Ölen, die weitgehend aus Ölen auf der Basis von Rizinolsäure mit Ethylenoxid bestehen.

2. Formulierung zur Verwendung nach Anspruch 1 für die Verabreichung von wenigstens einem Protein an ein Säugetier, wobei das wenigstens eine Protein ausgewählt ist aus der Gruppe bestehend aus Insulin, Parathyroidhormon, Parathyroid-ähnlichem Hormon, Glukagon, insulinotropem Hormon, Vasopressin und am reproduktiven System beteiligten Hormonen, Chemotaktinen; Cytokinen inklusive Interleukinen 1, 2 und RA, aber ohne die Interferone; Chemokinen; Enzymen inklusive Proteasen und Proteaseinhibitoren; Wachstumsfaktoren inklusive azidischen und basischen Fibroblastwachstumsfaktoren, epidermalem Wachstumsfaktor, Tumornekrosefaktoren, von Thrombozyten abgeleitem Wachstumsfaktor, Granulozyten-Makrophagen-Kolonie stimulierendem Faktor, Neuritenwachstumsfaktor und insulinähnlichem Wachstumsfaktor-1, Hormonen inklusive den Gonadotrophinen und Somatomedianen, Immunglobulinen, lipidbindenden Proteinen und löslichem CD4, Urokinase, Streptokinase, Superoxiddismutase (SOD), Katalase, Phenylalanin-Ammonia-Lyase, L-Asparaginase, Pepsin, Uricase, Trypsin, Chymotrypsin, Elastase, Carboxypeptidase, Lactase, Sucrase, ziliärem Neuritentransformationsfaktor (CNTF), Gerinnungsfaktor VIII, Erythropoietin, Thrombopoietin, Insulintropin, Cholezystokinin, Glukagonähnlichem Peptid I, intrinsischem Faktor, Ob-Gen-Produkt, Gewebeplasminogenaktivator (tPA), vom Gehirn stammendem Neuritfaktor, Phenylalanintransporter, Bürstensaumenzymen und Transportern, und zum Verstärken von Absorption, Verteilung und Freisetzung des wenigstens einen Proteins in oder an dem Säugetier, wobei die Formulierung aus dem wenigstens einen Protein in einer Mikroemulsion besteht, wobei die Mikroemulsion von einer Dispersion von Vesikeln oder Mikroschwämmen einer Komponente auf Fettsäurebasis in einem wässrigen oder anderen pharmakologisch akzeptablen Träger gebildet wird, in dem Distickstoffoxid aufgelöst ist, wobei die Komponente auf Fettsäurebasis wenigstens eine Substanz auf der Basis einer langkettigen Fettsäure umfasst, die ausgewählt ist aus der Gruppe bestehend aus freien Fettsäuren und Derivaten von freien Fettsäuren.

3. Formulierung nach Anspruch 1, wobei die Zusammensetzung auch das Antioxidans dl-α-Tocopherol oder ein stabiles Derivat davon enthält.

4. Formulierung nach Anspruch 1, wobei die Zusammensetzung einen Proteaseinhibitor enthält.

5. Formulierung nach Anspruch 1, wobei die Dispersion **dadurch gekennzeichnet ist, dass** wenigstens 50 % der Vesikel einen Durchmesser zwischen 80 Nanometern und 3 µm und der Mikroschwämme einen zwischen 1,5 und 6,0 µm haben.

6. Formulierung nach Anspruch 1 oder 2, wobei die Komponente auf Fettsäurebasis ausgewählt ist aus der Gruppe bestehend aus Oleinsäure, Linolsäure, Alpha-Linolensäure, Gamma-Linolensäure, Arachidonsäure, Eicosapentanoesäure [C20:5ω3], Decosahexanoesäure [C22:6ω3], und Rizinolsäure, und Derivaten davon, ausgewählt aus der Gruppe bestehend aus den C₁ bis C₆ Alkylestern davon, den Glycerol-Polyethylenglykolestern davon und dem Reaktionsprodukt von hydrierten und unhydrierten natürlichen Ölen, die weitgehend aus Ölen auf der Basis von Rizinolsäure mit Ethylenoxid bestehen.

7. Formulierung nach Anspruch 1, wobei die Fettsäurekomponente der Mikroemulsion durch das als Vitamin F Ethylester bekannte Gemisch von veresterten Fettsäuren gebildet wird.

8. Formulierung nach Anspruch 1, wobei die Dispersion in Form von Mikroschwämmen vorliegt und von sehr langkettigen polyungesättigten Fettsäuren gebildet wird, die aus Eicosapentanoesäure [C20:5ω3] und Decosahexanoesäure [C22:6ω3] oder einem Gemisch aus beiden ausgewählt sind.

9. Formulierung nach Anspruch 8, wobei die Fettsäurekomponente ferner das Reaktionsprodukt von hydrierten natürlichen Ölen beinhaltet, die weitgehend aus Ölen auf der Basis von Rizinolsäure mit Ethylenoxid oder modifizierten Derivaten davon bestehen.

10. Formulierung nach Anspruch 1, wobei das Protein Insulin ist.

## Revendications

1. Formulation à utiliser pour l'administration d'au moins une protéine thérapeutique mammalienne à un mammifère, et pour améliorer l'absorption, la distribution et la libération de la au moins une protéine thérapeutique mammalienne chez ou sur le mammifère, la formulation consistant en au moins une protéine thérapeutique mammalienne dans une microémulsion, laquelle microémulsion est constituée par une dispersion de vésicules ou de micro-éponges d'un composant à base d'acides gras dans un véhicule aqueux ou autre pharmacologiquement acceptable dans lequel de l'oxyde nitreux est dissous, le composant à base d'acides gras comprenant au moins une substance à base d'acides gras à chaîne longue sélectionnée parmi le groupe consistant en acides gras libres et dérivés d'acides gras libres, lesquels dérivés de ceux-ci sont sélectionnés parmi le groupe consistant en les esters alkyliques en C₁ à C₆ de ceux-ci, les esters de glycérol-polyéthylène glycol de ceux-ci et le produit de réaction d'huiles naturelles hydrogénées et non hydrogénées composées en grande partie d'huiles à base d'acide ricinoléique avec de l'oxyde d'éthylène.

2. Formulation à utiliser selon la revendication 1 pour l'administration à un mammifère d'au moins une protéine, dans laquelle la au moins une protéine est sélectionnée parmi le groupe consistant en insuline, hormone parathyroïde, hormone analogue à la parathyroïde, glucagon, hormone insulinotrophique, vasopressine et hormones impliquées dans le système reproductif, chimiotactines; cytokines y compris interleukines 1,2 et RA mais à l'exclusion des interférons; chimiokines; enzymes y compris protéases et inhibiteurs de protéases; facteurs de croissance y compris facteurs de croissance des fibroblastes acides et basiques, facteur de croissance épidermique, facteurs de nécrose tumorale, facteur de croissance d'origine plaquettaire, facteur stimulant les colonies de granulocytes-macrophages, facteur de croissance des neurites et facteur 1 de croissance analogue à l'insuline, hormones y compris les gonadotrophines et somatomédines, immunoglobulines, protéines de liaison aux lipides et CD4 soluble, urokinase, steptokinase, superoxyde dismutase (SOD), catalase, phénylalanine ammonialyase, L-asparaginase, pepsine, uricase, trypsine, chymotrypsine, élastase, carboxypeptidase, lactase, sucrase, facteur neurotrophique ciliaire (CNTF), facteur VIII de la coagulation, érythropoïétine, thrombopoïétine, insulinotropine, cholécystokinine, peptide 1 glucagon-like, facteur intrinsèque, produit génique Ob, activateur tissulaire du plasminogène (tPA), facteur neurotrophique dérivé du cerveau, transporteur de phénylalanine, enzymes et transporteurs de la bordure en brosse, et pour améliorer l'absorption, la distribution et la libération de la au moins une protéine chez ou sur le mammifère, la formulation consistant en au moins une protéine dans une microémulsion, laquelle microémulsion est constituée par une dispersion de vésicules ou de micro-éponges d'un composant à base d'acides gras dans un véhicule aqueux ou autre pharmacologiquement acceptable dans lequel de l'oxyde nitreux est dissous, le composant à base d'acides gras comprenant au moins une substance à base d'acides gras à chaîne longue sélectionnée parmi le groupe consistant en acides gras libres et dérivés d'acides gras libres.

3. Formulation selon la revendication 1, dans laquelle la composition contient aussi l'antioxydant dl-α-tocophérol ou un dérivé stable de celui-ci.

4. Formulation selon la revendication 1, dans laquelle la composition comprend un inhibiteur de protéases.

5. Formulation selon la revendication 1, dans laquelle la dispersion est **caractérisée en ce qu'**au moins 50 % des vésicules ont une taille de diamètre d'entre 80 nanomètres et 3 µm et celle des micro-éponges est d'entre 1,5 et 6,0 µm.

6. Formulation selon la revendication 1 ou 2, dans laquelle le composant à base d'acides gras est sélectionné parmi le groupe consistant en acide oléique, acide linoléique, acide alpha-linolénique, acide gamma-linolénique, acide arachidonique, acide éicosapentaénoïque [C20:5ω3], acide docosahexaénoïque [C22:6ω3] et acide ricinoléique, et les dérivés de ceux-ci sont sélectionnés parmi le groupe consistant en esters alkyliques en C₁ à C₆ de ceux-ci, les esters de glycérol-polyéthylène glycol de ceux-ci et le produit de réaction d'huiles naturelles hydrogénées et non hydrogénées composées en grande partie d'huiles à base d'acide ricinoléique avec de l'oxyde d'éthylène.

7. Formulation selon la revendication 1, dans laquelle le composant d'acides gras de la microémulsion est constitué par le mélange d'acides gras estérifiés connu comme un ester éthylique de vitamine F.

8. Formulation selon la revendication 1, dans laquelle la dispersion est sous la forme de micro-éponges et est constituée par des acides gras polyinsaturés à chaîne très longue sélectionnés parmi l'acide éicosapentaénoïque [C20:5ω3] et l'acide docosahexaénoïque [C22:6ω3] ou un mélange des deux.

9. Formulation selon la revendication 8, dans laquelle le composant d'acides gras comprend en outre le produit de réaction d'huiles naturelles hydrogénées composées en grande partie d'huiles à base d'acide ricinoléique avec de l'oxyde d'éthylène ou de dérivés modifiés de celles-ci.

10. Formulation selon la revendication 1, dans laquelle la protéine est de l'insuline.
